(19) Europäisches Patentamt
European Patent Office
Office européen des brevets

(11) EP 4 333 796 B1

(12) EUROPEAN PATENT SPECIFICATION

(45) Date of publication and mention of the grant of the patent:
**26.11.2025 Bulletin 2025/48**

(21) Application number: **21933634.4**

(22) Date of filing: **30.03.2021**

(51) International Patent Classification (IPC):
***A61K 8/06*** (2006.01) ***A61K 8/34*** (2006.01)
***A61K 8/365*** (2006.01) ***A61K 8/37*** (2006.01)
***A61K 31/375*** (2006.01) ***A61K 8/39*** (2006.01)
***A61K 8/67*** (2006.01) ***A61K 8/86*** (2006.01)
***A61Q 17/04*** (2006.01) ***A61Q 19/00*** (2006.01)
***A61Q 19/02*** (2006.01) ***A61Q 19/08*** (2006.01)

(52) Cooperative Patent Classification (CPC):
**A61Q 19/00; A61K 8/068; A61K 8/345; A61K 8/365; A61K 8/39; A61K 8/676; A61K 8/678; A61K 8/86; A61Q 17/04; A61Q 19/02; A61Q 19/08;**
A61K 2800/30; A61K 2800/31

(86) International application number:
**PCT/CN2021/084017**

(87) International publication number:
**WO 2022/204951 (06.10.2022 Gazette 2022/40)**

# (54) A COMPOSITION FOR CARING FOR KERATIN MATERIALS

ZUSAMMENSETZUNG ZUR PFLEGE KERATINHALTIGER MATERIALIEN

COMPOSITION DESTINÉE AU SOIN DES MATIÈRES KÉRATINIQUES

(84) Designated Contracting States:
**AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**13.03.2024 Bulletin 2024/11**

(73) Proprietor: **L'OREAL**
**75008 Paris (FR)**
Designated Contracting States:
**AT BE BG CH CY CZ DE DK EE ES FI FR GB GR HR HU IE IS IT LI LT LU LV MC MK MT NL NO PL PT RO RS SE SI SK SM TR**

(72) Inventors:
- **WU, Di**
  **Shanghai 201206 (CN)**
- **SUN, Lingling**
  **Shanghai 201206 (CN)**

(74) Representative: **Casalonga**
**Casalonga & Partners**
**Bayerstraße 71/73**
**80335 München (DE)**

(56) References cited:
**EP-A1- 0 729 746**
**WO-A1-2009/016064**
**US-A- 5 843 411**
**US-A1- 2009 292 015**
**WO-A1-2009/016064**
**AU-A- 8 945 598**
**US-A1- 2009 292 015**

- **BURKE JOHN: "Solubility Parameters: Theory and Application", BOOK AND PAPER GROUP ANNUAL, VOLUME 3 (1984), 1 September 1984 (1984-09-01), Austin, Texas, XP093293038, Retrieved from the Internet <URL:https://cool.culturalheritage.org/coolaic/sg/bpg/annual/v03/bp03-04.html>**
- **ANONYMOUS: "Benton Cleasing water", COSDNA, 21 February 2018 (2018-02-21), XP055974732, Retrieved from the Internet <URL:http://www.cosdna.com/chs/cosmetic_cae0341566.html> [retrieved on 20221025]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- ANONYMOUS: "Celery Biovitamin CE Complex Repair Serum", YANTAI CELLREGNERAT BIOTECHNOLOGY CO., LTD., 29 March 2018 (2018-03-29), XP055974731, Retrieved from the Internet <URL:http://ftba.nmpa.gov.cn:8181/ftban/itownet/hzp_ba/fw/pz.jsp?processid=20180327181906e9tzu&nid=20180327181906e9tzu> [retrieved on 20221025]
- ANONYMOUS: "Senser Ferulic Acid Serum", YANTAI CESTCA TRADING CO., LTD., 21 March 2020 (2020-03-21), XP055974728, Retrieved from the Internet <URL:http://ftba.nmpa.gov.cn:8181/ftban/itownet/hzp_ba/fw/pz.jsp?processid=20200611092004fa6bn&nid=20200611092004fa6bn> [retrieved on 20221025]
- ANONYMOUS: "Sense L-Vitamin C Serum", YANTAI CESTCA TRADING CO., LTD., 20 April 2018 (2018-04-20), XP055974724, Retrieved from the Internet <URL:http://ftba.nmpa.gov.cn:8181/ftban/itownet/hzp_ba/fw/pz.jsp?processid=20180404085807d0uo1&nid=20180404085807d0uo1> [retrieved on 20221025]
- ANONYMOUS: "Basiclab Serum Ascorbic Acid", COSDNA, 28 July 2020 (2020-07-28), XP055974723, Retrieved from the Internet <URL:http://www.cosdna.com/chs/cosmetic_0573521988.html> [retrieved on 20221025]

## Description

### TECHNICAL FIELD

The present invention relates to a composition for caring for keratin materials. The present invention also relates to a non-therapeutic process for caring for keratin materials.

### BACKGROUND

Ascorbic acid (vitamin C) is a strong antioxidant active ingredient, stimulates the synthesis of connective tissue, in particular of collagen, enhances the defense of skin tissue against damage by environmental agents such as ultraviolet radiation and pollution, compensates for any vitamin E deficiency of the skin, depigments the skin and has an anti-free radical function. The last two properties make it an excellent candidate as a cosmetic or dermatological active agent for combating and/or preventing skin ageing.

Ferulic acid is an antioxidant active agent and can provide whitening/lightening, anti-inflammation and UV protection benefits.

It is desired to formulate products comprising ascorbic acid or ferulic acid in the cosmetic and dermatological fields.

However, formulating such products face huge challenges.

Because of its chemical structure (alpha-ketolactone), ascorbic acid is very sensitive to certain environmental factors such as oxygen and water. The rapid degradation of ascorbic acid occurs in the presence of oxygen and water, which cause decreased potency and color change issue.

Ferulic acid will undergoes a decarboxylation reaction resulting in a loss in the stability of ferulic acid and an unpleasant odor when contacting with water after several days, for example 7 days when the content of ferulic acid is no less than 1.9 wt.%, 15 days when the content of ferulic acid is no less than 1.5 wt.%, and 30 days when the content of ferulic acid is no less than 1 wt.%, relative to the composition containing it.

Some products focus on water-free cosmetic base to avoid contacting with water. For example, there are some two-part products, wherein the cosmetic active ingredients such as ascorbic acid and ferulic acid present in the form of anhydrous powder (see e.g. EP0729746A1). However, bioavailability of the active ingredient is a challenge for some products.

There is still a need for cosmetic products comprising active ingredients such as ascorbic acid or ferulic acid, which is stable and presents bioavailability of cosmetic active ingredients.

### SUMMARY OF THE INVENTION

Thus, one object of the present is thus to provide a composition for caring for keratin materials comprising cosmetic active ingredients sensitive to oxygen and/or water, which is stable, i.e. the cosmetic active ingredient therein will not substantially decompose.

Another object of the present is thus to provide a composition for caring for keratin materials comprising cosmetic active ingredients sensitive to oxygen and/or water, which presents bioavailability of cosmetic active ingredients.

Still another object of the present invention is to provide a non-therapeutic process for caring for keratin materials.

Thus, according to one aspect, the present invention as defined in the appended claims provides an anhydrous composition for caring for keratin materials comprising:

a) at least one cosmetic active ingredient sensitive to water and/or oxygen;
b) at least one non-ionic surfactant selected from polyglyceryl fatty acid esters with a HLB value not less than 13 at the temperature of 25 °C and ethoxylated/propoxylated fatty alcohols; and
c) dipropylene glycol;

wherein the composition does not comprise any cationic surfactant.

The anhydrous composition according to the present invention is stable for at least 2 months.

In the present application, the stability is evaluated by testing the degradation of cosmetic active ingredient.

Surprisingly, the inventors found that the anhydrous composition can form a microemulsion when mixed with water or a hydrous system by gentle mixing, so as to enhance the bioavailability of the cosmetic active ingredient.

The hydrous system can be any system comprising no less than 40 wt. % of water, relative to the total weight of the system.

According to another aspect, the present invention provides a non-therapeutic process for caring for keratin materials, comprising:

i) mixing the anhydrous composition according to the present invention and water or a hydrous system to form a microemulsion; and
ii) applying the microemulsion to the keratin materials.

Other subjects and characteristics, aspects and advantages of the invention will emerge even more clearly on reading the description and the examples that follow.

## BRIEF DESCRIPTION OF THE DRAWINGS

Implementations of the present invention will now be described, by way of example only, with reference to the attached figures, wherein:

Fig.1 shows the Raman spectra of 400 - 2000cm$^{-1}$ for Ferulic Acid.
Fig.2 shows the Raman spectra of 400 - 2000cm$^{-1}$ for Tocopherol.
Fig. 3 shows the penetration profile of ferulic acid and tocopherol for the composition of invention example 5 after mixing with 48 wt. % water, relative to the total weight of the obtained mixture, wherein FA means ferulic acid, and VE means tocopherol.
Fig.4 shows the penetration profile of ferulic acid and Tocopherol for the composition of comparative example 4, wherein FA means ferulic acid, and VE means tocopherol.

## DETAILED DESCRIPTION OF THE INVENTION

Unless otherwise defined, all technical and scientific terms used herein have the same meaning as commonly understood by those skilled in the art the present invention belongs to. When the definition of a term in the present description conflicts with the meaning as commonly understood by those skilled in the art the present invention belongs to, the definition described herein shall apply.
In that which follows and unless otherwise indicated, the limits of a range of values are included within this range, in particular in the expressions "between...and..." and "from ... to ...".
Moreover, the expression "at least one" used in the present description is equivalent to the expression "one or more".
Throughout the instant application, the term "comprising" is to be interpreted as encompassing all specifically mentioned features as well optional, additional, unspecified ones. As used herein, the use of the term "comprising" also discloses the embodiment wherein no features other than the specifically mentioned features are present (i.e. "consisting of").
Unless otherwise specified, all numerical values expressing amount of ingredients and the like which are used in the description and claims are to be understood as being modified by the term "about". Accordingly, unless indicated to the contrary, the numerical values and parameters described herein are approximate values which are capable of being changed according to the desired purpose as required.
All percentages in the present invention refer to weight percentage, unless otherwise specified.
For the purposes of the present invention, the term "keratin materials" is intended to cover human skin, mucous membranes such as the lips. Facial skin is most particularly considered according to the present invention.
By "anhydrous", it means that no water is added on purpose and the oil content of the composition is less than 0.5% by weight, relative to the total weight of the composition. In particular, there is no water in the composition.
The anhydrous composition according to the present invention comprises:

a) at least one cosmetic active ingredient sensitive to water and/or oxygen;
b) at least one non-ionic surfactant selected from polyglyceryl fatty acid esters with a HLB value not less than 13 at the temperature of 25 °C and ethoxylated/propoxylated fatty alcohols; and
c) dipropylene glycol;

wherein the composition does not comprise any cationic surfactant.

## Cosmetic active ingredients

According to the first aspect, the anhydrous composition according to the present invention comprises at least one cosmetic active ingredient sensitive to water and/or oxygen selected from ferulic acid, ascorbic acid and tocopherols.

Ferulic acid

Ferulic acid, CAS number of which is 1135-24-6, is also called 4-Hydroxy-3-methoxycinnamic acid, which has the following formula:

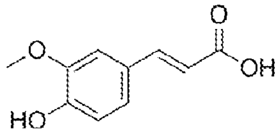

Ferulic acid can be broadly found in giant fennel, the seeds of coffee, apple, artichoke, peanut, and orange, as well as in both seeds and cell walls of commelinid plants (such as rice, wheat, oats, and pineapple). Like many natural phenols, it is a strong antioxidant that is very reactive toward free radicals and reduces oxidative stress. Many studies suggest that ferulic acid may have antitumor activity.
Mention may be made of such product, for example, ORYZA FERULIX from the company ORYZA OIL & FAT CHEMICAL.
If presents, ferulic acid is present in an amount ranging from 0.01 wt.% to 5 wt.%, preferably from 0.1 wt.% to 1.9 wt.%, relative to the total weight of the composition.

Ascorbic acid

Ascorbic acid has the following structural formula:

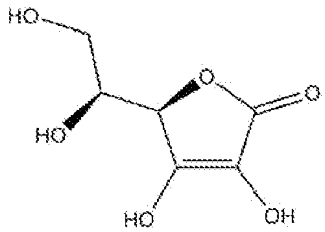

Ascorbic acid can be extracted from various vegetable sources in which it occurs naturally, such as rose hips, blackcurrants, the juice of citrus fruits, and the ripe fruit of Capsicum annuum L.
A common synthetic procedure involves the hydrogenation of D-glucose to D-sorbitol, followed by oxidation using Acetobacter suboxydans to form L-sorbose. A carboxyl group is then added at C1 by air oxidation of the diacetone derivative of Lsorbose and the resulting diacetone-2-keto-L-gulonic acid is converted to L-ascorbic acid by heating with hydrochloric acid.
As a commercial product of ascorbic acid, mention can be made of the product sold under the trade name YSA-SALICYLIC ACID PHARMACEUTICAL GRADE from the company NOVACYL.
If presents, ascorbic acid is present in an amount ranging from 1 wt.% to 15 wt.%, preferably from 8 wt.% to 12 wt.%, relative to the total weight of the composition.

Tocopherols

Tocopherols, also called as Vitamin E, have the following structure of formula (I):

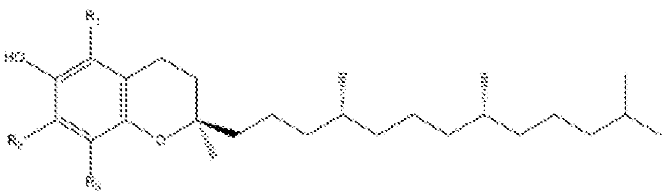
(I),

wherein

- $R_1$ is selected from hydrogen and methyl;
- $R_2$ is selected from hydrogen and methyl; and

$R_3$ is methyl.

There are four tocopherols (α, β, γ, and δ-tocotrienol).

α-tocotrienol has the following structure:

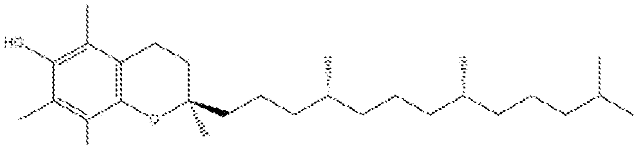

,

i.e. in formula (I), $R_1$ is methyl, $R_2$ is methyl, R3 is methyl.

β-tocotrienol has the following structure:

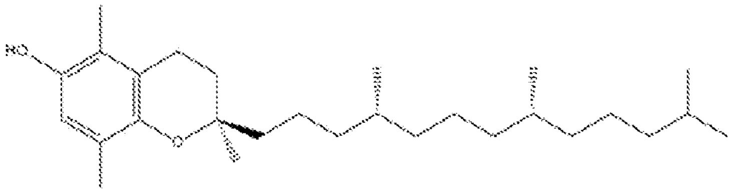

i.e. in formula (I), $R_1$ is methyl, $R_2$ is hydrogen, R3 is methyl.

γ-tocotrienol has the following structure:

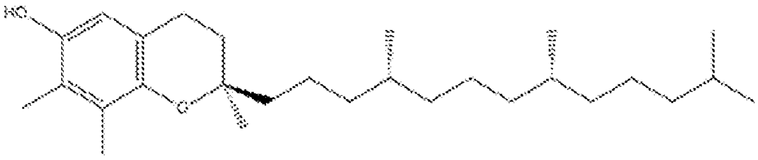

.

i.e. in formula (I), $R_1$ is hydrogen, $R_2$ is methyl, R3 is methyl.

δ-tocotrienol has the following structure:

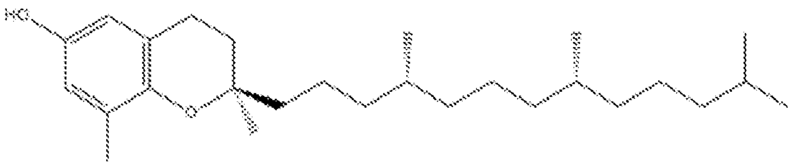

i.e. in formula (I), $R_1$ is hydrogen, $R_2$ is hydrogen, R3 is methyl.

α-Tocopherol is the only form of vitamin E that is actively maintained in the human body and thus is the form of vitamin E found in the greatest quantities in the blood and tissues.

As a commercial product of tocopherol, mention can be made of the product sold under the trade name DL ALPHA TOCOPHEROL (0410276) from the company DSM

NUTRITIONAL PRODUCTS.

If presents, tocopherol is present in an amount ranging from 0.1 wt.% to 10 wt.%, preferably from 0.1 wt.% to 5 wt.%, relative to the total weight of the composition.

If tocopherol is present as the cosmetic active ingredient, preferably, the anhydrous composition further comprises an oil selected from undecane, tridecane, C15-19 alkane, isohexadecane, dicaprylyl ether, hydrogenated polyisobutene, squalane in an amount ranging from 0.1 wt.% to 5 wt.%, preferably from 0.5 wt.% to 2 wt.%, relative to the total weight of the composition.

## Non-ionic surfactants

According to the first aspect, the composition according to the present invention comprises at least one non-ionic

surfactant selected from polyglyceryl fatty acid esters with a HLB value not less than 13 at the temperature of 25 °C and ethoxylated/propoxylated fatty alcohols.

**Polyglyceryl fatty acid ester with a HLB value not less than 13**

The HLB (hydrophile-lipophile balance) value is defined according to Griffin's method in J. Ploughshare. Cosm. Chem. 1954 (volume 5), pages 249-256. In particular, "HLB" value relates to the ratio of hydrophilic groups and lipophilic groups in surfactants, and also relates to solubility of the surfactants. Higher HLB surfactants are more soluble in hydrophilic system.

Preferably, the polyglyceryl fatty acid ester is selected from mono-, di- and tri-esters of glycerin and a saturated or unsaturated fatty acid including 8 to 12 carbon atoms, such as lauric acid, capric acid, and caprylic acid.

Preferably, the polyglyceryl fatty acid ester is selected from the group consisting of PG2 caprate, PG2 dicaprate, PG2 tricaprate, PG2 caprylate, PG2 dicaprylate, PG2 tricaprylate, PG2 laurate, PG2 dilaurate, PG2 trilaurate, PG3 caprate, PG3 dicaprate, PG3 tricaprate, PG3 caprylate, PG3 dicaprylate, PG3 tricaprylate, PG3 laurate, PG3 dilaurate, PG3 trilaurate, PG4 caprate, PG4 dicaprate, PG4 tricaprate, PG4 caprylate, PG4 dicaprylate, PG4 tricaprylate, PG4 laurate, PG4 dilaurate, PG4 trilaurate, PG5 caprate, PG5 dicaprate, PG5 tricaprate, PG5 caprylate, PG5 dicaprylate, PG5 tricaprylate, PG5 laurate, PG5 dilaurate, PG5 trilaurate, PG6 caprate, PG6 dicaprate, PG6 tricaprate, PG6 caprylate, PG6 dicaprylate, PG6 tricaprylate, PG6 laurate, PG6 dilaurate, PG6 trilaurate, PG7 caprate, PG7 dicaprate, PG7 tricaprate, PG7 caprylate, PG7 dicaprylate, PG7 tricaprylate, PG7 laurate, PG7 dilaurate, PG7 trilaurate, PG8 caprate, PG8 dicaprate, PG8 tricaprate, PG8 caprylate, PG8 dicaprylate, PG8 tricaprylate, PG8 laurate, PG8 dilaurate, PG8 trilaurate, PG9 caprate, PG9 dicaprate, PG9 tricaprate, PG9 caprylate, PG9 dicaprylate, PG9 tricaprylate, PG9 laurate, PG9 dilaurate, PG9 trilaurate, PG10 caprate, PG10 dicaprate, PG10 tricaprate, PG10 caprylate, PG10 dicaprylate, PG10 tricaprylate, PG10 laurate, PG10 dilaurate, and PG10 trilaurate.

It is more preferable that the polyglyceryl fatty acid ester is selected from polyglyceryl caprylate with a polyglyceryl moiety derived from 2 to 10 glycerol units.

As examples of polyglyceryl caprylate with a polyglyceryl moiety derived from 2 to 10 glycerol units, mention can be made of polyglyceryl-6 caprylate, for example, a product sold by the company TAIYO KAGAKU under the name SUNSOFT Q-8H-C.

If presents, the polyglyceryl fatty acid ester is present in an amount ranging from 0.5 wt.% to 10 wt.%, preferably from 1 wt.% to 5 wt.%, and more preferably from 1 wt.% to 4 wt.%, relative to the total weight of the composition.

Ethoxylated/propoxylated fatty alcohols

Ethoxylated/propoxylated fatty alcohols are ethers formed from the reaction of a fatty alcohol with propylene oxide and ethylene oxide.

Advantageously, the fatty alcohol is selected from linear or branched C8-C30 fatty alcohol, preferably C14-C22 fatty alcohol.

Preferably, the ethoxylated/propoxylated fatty alcohol is selected from ethoxylated/propoxylated C14-C22 fatty alcohols.

Preferably, the ethoxylated/propoxylated fatty alcohol is selected from ethoxylated/propoxylated C14-C22 fatty alcohols with 1-40 EO (ethylene oxide) unit and 1-40 PO (propylene oxide) unit.

Examples of ethoxylated/propoxylated fatty alcohols include PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9, PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-Laureth-5, PPG-6-Laureth-3, PPG-25-Laureth-25, PPG-3-Myreth-3, PPG-3-Myreth-11, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

If presents, the ethoxylated/propoxylated fatty alcohol is present in an amount ranging from 1 wt.% to 10 wt.%, and preferably from 2 wt.% to 8 wt.%, relative to the total weight of the composition.

## Dipropylene glycol

According to the first aspect, the composition according to the present invention comprises dipropylene glycol.
In the composition according to the present invention, dipropylene glycol is used as a co-surfactant and a solvent.
Advantageously, dipropylene glycol is present in an amount ranging from 5 wt.% to 30 wt.%, preferably from 5 wt.% to 20 wt.%, relative to the total weight of the composition.

## C3-C6 polyol

In some embodiments, the composition according to the present invention further comprises a C3-C6 polyol other than dipropylene glycol. Preferably, the C3-C6 polyol is selected from C3-C6 glycol and glycerine.
As examples of C3-C6 glycol other than dipropylene glycol, mention can be made of propylene glycol, butylene glycol, pentylene glycol, hexylene glycol, PEG-6, and PEG-8.
In the present invention, the definition of glycol includes all possible isomer. For example, propylene glycol includes 1,3-propylene glycol, 1,2-propylene glycol and 1,1-propylene glycol. Butylene glycol includes 1,4-butylene glycol, 1,3-butylene glycol, 1,2-butylene glycol, etc.
Preferably, the anhydrous composition according to the present invention comprises propylene glycol.
If presents, the C3-C6 polyol other than dipropylene glycol is present in an amount ranging from 1 wt.% to 50 wt.%, preferably from 10 wt.% to 40 wt.%, relative to the total weight of the composition.

## Additional cosmetic active agent(s)

Depend on the final purpose, the composition can comprise an additional cosmetic active agent(s).
As additional cosmetic active agents that may be used in the composition of the present invention, examples that may be mentioned include enzymes; flavonoids; moisturizers; anti-inflammatory agents; depigmenting agents; whitening agents (such as phenylethyl resorcinol);$\alpha$-hydroxy acids; $\beta$-hydroxy acids (such as capryloyl salicylic acid); retinoids; antibacterial active agents; tensioning agents; ceramides; essential oils; UV-screening agents (or sunscreens), and mixtures thereof.
It is easy for the skilled in the art to adjust the amount of the additional cosmetic active agent based on the final use of the composition according to the present invention.

## Additional adjuvants or additives

The composition according to the present invention may also contain conventional cosmetic adjuvants or additives, for instance fragrances, preserving agents and bactericides, opacifiers, dyes, softeners, buffers, electrolytes such as sodium chloride, or a pH regulator (for example citric acid or potassium hydroxide), and mixtures thereof.
Needless to say, the skilled in the art will take care to select the optional adjuvant(s) added to the composition according to the present invention such that the advantageous properties intrinsically associated with the composition according to the present invention are not, or are not substantially, adversely affected by the envisaged addition.
According to a preferred embodiment, the present invention provides an anhydrous composition for caring for keratin materials comprising, relative to the total weight of the composition:

a) at least one cosmetic active ingredient selected from ferulic acid, ascorbic acid and tocopherols;
b) from 1 wt.% to 4 wt.% of at least one non-ionic surfactant selected from polyglyceryl caprylate with a polyglyceryl moiety derived from 2 to 10 glycerol units and ethoxylated/propoxylated C14-C22 fatty alcohols with 1-40 EO units and 1-40 PO units; and
c) from 5 wt.% to 20 wt.% of dipropylene glycol;

wherein the composition does not any cationic surfactant.

## Galenic form and use

The composition according to the present invention can be an anhydrous product. Consumers can mix it with water or a hydrous system to obtain a microemulsion.
Alternatively, the composition according to the present invention can be one part of a dual-chamber product,

wherein the composition according to the present invention is in one chamber, a homogeneous hydrous formulation is in the other chamber. Consumers can mix the composition in one chamber with the homogeneous hydrous formulation in the other chamber to obtain a microemulsion.

The "microemulsion" may be defined in two ways, namely, in a broader sense and in a narrower sense. That is to say, there are one case ("microemulsion in the narrow sense" ) in which the microemulsion refers to a thermodynamically stable isotropic single liquid phase containing a ternary system having three ingredients of an oily component, an aqueous component and a surfactant, and the other case ("microemulsion in the broad sense" ) in which among thermodynamically unstable typical emulsion systems the microemulsion additionally includes those such emulsions presenting transparent or translucent appearances due to their smaller particle sizes (Satoshi Tomomasa, et al., Oil Chemistry, Vol. 37, No. 11 (1988) , pp. 48-53). The "microemulsion" as used herein refers to a "microemulsion in the broad sense".

Advantageously, in the microemulsion formed with the composition according to the present invention, the droplets have a number-average size of 120 nm or less, preferably from 20 nm to 80 nm, more preferably from 20 nm to 50 nm.

The volume-average size of the droplets may be determined in particular according to the known method of dynamic light scattering (DLS). By way of apparatus that can be used for this determination, mention may be made of the particle size analyser of the brand Malvern, model Zetasizer Nano ZS, equipped with a standard laser having a power of 4 mW, and at a wavelength of 633 nm. This device is also equipped with a correlator (25 ns to 8000 s, 4000 channels max.).

The microemulsion obtained by the anhydrous composition with water or a hydrous system can be used to care for keratin materials

Thus, according to the second aspect, the present invention provides a non-therapeutic process for caring for keratin materials, comprising:

i) mixing the anhydrous composition according to the present invention and water or a hydrous system to form a microemulsion; and
ii) applying the microemulsion to the keratin materials.

The hydrous system can be any system comprising no less than 40 wt.% of water, relative to the total weight of the system.

The examples that follow are aimed at illustrating the compositions according to the present invention.

## EXAMPLES

Main raw materials used, trade names and supplier thereof are listed in Table 1.

Table 1

| INCI Name | Trade Name | Supplier |
|---|---|---|
| POLYGLYCERYL-6 CAPRYLATE | SUNSOFT Q-8H-C | TAIYO KAGAKU |
| DIPROPYLENE GLYCOL | DIPROPYLENE GLYCOL LO | DOW |
| FERULIC ACID | ORYZA FERULIX | ORYZA OIL & FAT CHEMICAL |
| ASCORBIC ACID | YSA-SALICYLIC ACID PHARMACEUTICAL GRADE | NOVACYL |
| TOCOPHEROL | DL ALPHA TOCOPHEROL (0410276) | DSM NUTRITIONAL PRODUCTS |
| PROPANEDIOL | ZEMEA PROPANEDIOL | DUPONT TATE AND LYLE BIO PRODUCTS |
| PROPYLENE GLYCOL | MONOPROPYLENE GLYCOL | DONGYING HI-TECH SPRING CHEMICAL IN |
| POLYGLYCERYL-2 OLEATE | SUNSOFT Q-17D(G)-C | TAIYO KAGAKU |
| PPG-5-CETETH-20 | PROCETYL AWS-LQ-(AP) | CRODA |

## Invention examples 1-5 and comparative examples 1-3

Compositions according to invention examples (IE) 1-5 and comparative examples(CE) 1-3 were prepared according to the contents given in Table 2 (the contents are expressed as weight percentages of active material relative to

the total weight of each composition, unless otherwise indicated).

Table 2

| Components | IE 1 | IE 2 | IE 3 | CE 1 | CE 2 | CE 3 | CE 4 | IE.4 | IE.5 |
|---|---|---|---|---|---|---|---|---|---|
| POLYGLYCERYL-6 CAPRYLATE | 1 | 1.92 | 4 | - | - | 4 | - | 1.92 | - |
| DIPROPYLENE GLYCOL | 10 | 19.23 | 10 | 10 | 10 | 10 | 10 | 19.23 | 19.23 |
| FERULIC ACID | 1.9 | 3.65 | 1.9 | 1.9 | 1.9 | 1.9 | 1.9 | 3.65 | 3.65 |
| ASCORBIC ACID | - | - | - | - | - | - | 5 | 9.62 | 9.62 |
| TOCOPHEROL | - | - | - | - | - | - | 1 | 0.19 | 1.92 |
| PROPANEDIOL (1,3-PROPYLENE GLYCOL) | 20 | 38.46 | 20 | 20 | 20 | 20 | 20 | 38.46 | 38.46 |
| PROPYLENE GLYCOL (1,2-PROPYLENE GLYCOL) | QS100 | QS100 | QS100 | QS100 | QS100 | QS100 | 19.42 | QS100 | QS100 |
| POLYGLYCERYL-2 OLEATE | - | - | - | - | 4 | - | - | - | - |
| PPG-5-CETETH-20 | - | - | - | - | - | - | 1 | - | 7.7 |
| WATER | - | - | - | - | - | 50 | Qs10 0 | - | - |

**Preparation procedure:**

The compositions were prepared as follows, taking the composition of invention example 1 as an example:

1). introducing FERULIC ACID into DIPROPYLENE GLYCOL with gentle stirring until no visible particles, to obtain a mixture;
2). introducing POLYGLYCERYL-6 CAPRYLATE into the mixture and keep stirring; and
3). introducing 1,3-PROPYLENE GLYCOL and 1,2-PROPYLENE GLYCOL to obtain the composition by stirring.

**Evaluation:**

1. Stability

The stability of each composition obtained in invention examples 1-5 and comparative examples 1-3 was evaluated as follows.
After the preparation, the stability of each composition was determined by perceiving whether there is odor issue and observing whether there is color change after each composition was placed at room temperature (20°C) for 2 months.
If there is no odor issue and there is color change for a composition after the composition was placed at room temperature (20°C) for 2 months, then the composition passes the stability test. If there is odor issue and/or there is color change for a composition after the composition was placed at room temperature (20°C) for 2 months, then the composition fails the stability test.
The results were listed in Table 3.

2. Particle size

Each composition prepared in invention example 1-5 and comparative examples 1-3 were mixed with water by gentle mixing, then the particle size of droplets in the obtained mixtures were determined through Brookhaven DLS with scattering angle (°) of 90 and count rate of 355.1 kcps. The following criterion was used to evaluate the stability.
If particle size (Mean Diamater by Volume) is 120 nm or more, then no microemulsion is formed.

If particle size (Mean Diamater by Volume) is more than 10 nm and less than 120 nm, then a microemulsion is formed.

Whether a microemulsion was formed is listed in Table 3.

Table 3

| | Properties | IE.1 | IE.2 | IE.3 | CE.1 | CE.2 | CE.3 | CE.4 | IE.4 | IE.5 |
|---|---|---|---|---|---|---|---|---|---|---|
| Stability | Odor issue | No | No | No | No | No | Yes | Yes | No | No |
| | Color change | No | No | No | No | No | Yes | Yes | No | No |
| | Result | Pass | Pass | Pass | Pass | Pass | Fail | Fail | Pass | Pass |
| Microemulsion | | Yes | Yes | Yes | No | No | Yes | No | Yes | Yes |

It was found that the particle sizes of all droplets in each composition of invention examples 1-5 are within 40 nm.

3. Bioavailability

The penetration of cosmetic active ingredients in each composition prepared in Example 1 was characterized as follows.

**i) Preparation of skin tissue samples**

6ul of each composition to be tested was applied evenly on 0.8cm X 0.8cm porcine skin (pig ear skin from food industry), corresponding to 9 mg/cm$^2$. The porcine skin sample was then emerged on insert membrane with PBS underneath, followed by 37°C incubation at 95% RH for 2 hours. Treated sample was embedded in an OCT Tissue Freezing Medium, then frozen and cryo-sectioned into 20 μm thickness. It was further placed on a $CaF_2$ substrate for Raman confocal scanning. Three porcine samples were prepared for each composition. A Raman confocal mapping was acquired of each treated sample.

**ii) Raman spectroscopy**

A LabRam HR Evolution (Horiba Jobin-Yvon, Villeneuve-d'Ascq, France) Raman confocal microscope was used. Raman spectrum was obtained using a 532 nm DPSS laser with a power of 8 mW on the sample, coupled with a ×50 LM Plan objective (Olympus, NA 0.75, Rungis, France). The confocal hole was set at 100μm diameter for all measurements. The system was spectrally calibrated to the 520.7 cm$^{-1}$ spectral line of silicon before the test. Detection was facilitated by dispersing Raman-shifted radiation onto a charge-coupled device (CCD) detector using a grating of 600 lines/mm.

For pure cosmetic active ingredients, single point spectra were acquired with 25% laser intensity and 10 seconds acquisition time, for 400 - 2000cm$^{-1}$ spectral range.

For mapping in the sample evaluation, the step size was 3μm in both X and Y direction. The acquisition areas were 18 X 150 μm. For each spot, 50% laser intensity and 5 seconds acquisition time per spectrum was used. Spectral range was 400 - 2000cm$^{-1}$.

**iii) Data analysis**

Non-negative constrained least square (NCLS) analysis was performed using Matlab. Before statistical analysis, Raman spectra were subjected to a linear baseline correction. Cosmetic active ingredient spectral of 400 - 2000cm$^{-1}$ fingerprints was used for analysis.

Fig.1 shows the Raman spectra of 400 - 2000cm$^{-1}$ for Ferulic Acid.

Fig.2 shows the Raman spectra of 400 - 2000cm$^{-1}$ for Tocopherol.

A simplified description of the NCLS outcome can be defined as:

$$Ss = (SR1*C1) + (SR2*C2) + \ldots\ldots + (SRi*Ci) + R*CR$$

Ss: Acquired Raman signal of one pixel on treated porcine skin sample;
SRi: Raman signal of each suppositional ingredient (PCA component) in untreated porcine;

R: Raman signal of pure cosmetic active ingredient;
Ci: coefficient of each suppositional ingredient (PCA component) in the exact pixel;
CR: coefficient of cosmetic active ingredient in the exact pixel;

The computational co-efficient index of active ingredients can be used to generate the distribution profile of active ingredients from outer skin stratum corneum to the deeper dermis part.

Fig. 3 shows the penetration profile of ferulic acid and tocopherol for the composition of invention example 5 after mixing with 48 wt. % water, relative to the total weight of the obtained mixture, wherein FA means ferulic acid, and VE means tocopherol.

Fig. 4 shows the penetration profile of ferulic acid and Tocopherol for the composition of comparative example 4, wherein FA means ferulic acid, and VE means tocopherol.

It can be seen from the comparison of Fig. 3 and Fig.4 that for the composition according to invention example 5, tocopherol and ferulic acid have deeper penetration, compared to the composition of comparative example 4.

## Claims

1. An anhydrous composition for caring for keratin materials comprising:

    a) at least one cosmetic active ingredient sensitive to water and/or oxygen selected from ferulic acid, ascorbic acid and tocopherols;
    b) at least one non-ionic surfactant selected from polyglyceryl fatty acid esters with a HLB value not less than 13 at the temperature of 25 °C and ethoxylated/propoxylated fatty alcohols; and
    c) dipropylene glycol;

    wherein the composition does not comprise any cationic surfactant.

2. The composition according to claim 1, wherein the polyglyceryl fatty acid ester is selected from mono-, di- and tri-esters of glycerin and a saturated or unsaturated fatty acid including 8 to 12 carbon atoms.

3. The composition according to claim 1, wherein the polyglyceryl fatty acid ester is selected from polyglyceryl caprylate with a polyglyceryl moiety derived from 2 to 10 glycerol units.

4. The composition according to any of claims 1-3, wherein the ethoxylated/propoxylated fatty alcohol is selected from ethoxylated/propoxylated C14-C22 fatty alcohols.

5. The composition according to claim 4, wherein the ethoxylated/propoxylated fatty alcohol is selected from ethoxylated/propoxylated C14-C22 fatty alcohols with 1-40 EO units and 1-40 PO units.

6. The composition according to any of claims 1-5, wherein the polyglyceryl fatty acid ester is present in an amount ranging from 0.5 wt.% to 10 wt.%, preferably from 1 wt.% to 5 wt.%, and more preferably from 1 wt.% to 4 wt.%, relative to the total weight of the composition.

7. The composition according to any of claims 1-6, wherein the ethoxylated/propoxylated fatty alcohol is present in an amount ranging from 1 wt. % to 10 wt. %, and preferably from 2 wt. % to 8 wt. %, relative to the total weight of the composition.

8. The composition according to any of claims 1-7, wherein diropylene glycol is present in an amount ranging from 5 wt. % to 30 wt. %, preferably from 5 wt. % to 20 wt. %, relative to the total weight of the composition.

9. The composition according to any of claims 1-8, wherein the composition further comprises a C3-C6 polyol other than dipropylene glycol selected from glycerine, propylene glycol, butylene glycol, pentylene glycol, and hexylene glycol.

10. The composition according to claim 9, wherein the C3-C6 polyol other than dipropylene glycol is present in an amount ranging from 1 wt.% to 50 wt.%, preferably from 10 wt.% to 40 wt.%, relative to the total weight of the composition.

11. The composition according to claim 1, comprising, relative to the total weight of the composition:

a) at least one cosmetic active ingredient selected from ferulic acid, ascorbic acid and tocopherols;
b) from 1 wt.% to 4 wt.% of at least one non-ionic surfactant selected from polyglyceryl caprylate with a polyglyceryl moiety derived from 2 to 10 glycerol units and ethoxylated/propoxylated C14-C22 fatty alcohols with 1-40 EO units and 1-40 PO units; and
c) from 5 wt.% to 20 wt.% of dipropylene glycol;

wherein the composition does not comprise any cationic surfactant.

**12.** A non-therapeutic process for caring for keratin materials, comprising:

i) mixing the anhydrous composition according to any of claims 1-11 and water or a hydrous system to form a microemulsion; and
ii) applying the microemulsion to the keratin materials.

**Patentansprüche**

**1.** Wasserfreie Zusammensetzung zur Pflege keratinhaltiger Materialien, umfassend:

a) wenigstens einen kosmetischen Wirkstoff, der wasser- und/oder sauerstoffempfindlich ist, ausgewählt aus Ferulasäure, Ascorbinsäure und Tocopherolen;
b) wenigstens ein nichtionisches Tensid, ausgewählt aus Polyglycerylfettsäureestern mit einem HLB-Wert von nicht weniger als 13 bei einer Temperatur von 25 °C und ethoxylierten/propoxylierten Fettalkoholen; und
c) Dipropylenglykol;

wobei die Zusammensetzung kein kationisches Tensid umfasst.

**2.** Zusammensetzung nach Anspruch 1, wobei der Polyglycerylfettsäureester aus Mono-, Di- und Triestern von Glycerin und einer gesättigten oder ungesättigten Fettsäure mit 8 bis 12 Kohlenstoffatomen ausgewählt ist.

**3.** Zusammensetzung nach Anspruch 1, wobei der Polyglycerylfettsäureester aus Polyglycerylcaprylat mit einer Polyglyceryl-Komponente, die von 2 bis 10 Glycerineinheiten abgeleitet ist, ausgewählt ist.

**4.** Zusammensetzung nach einem der Ansprüche 1 - 3, wobei der ethoxylierte/propoxylierte Fettalkohol aus ethoxylierten/propoxylierten C14-C22-Fettalkoholen ausgewählt ist.

**5.** Zusammensetzung nach Anspruch 4, wobei der ethoxylierte/propoxylierte Fettalkohol aus ethoxylierten/propoxylierten C14-C22-Fettalkoholen mit 1-40 EO-Einheiten und 1-40 PO-Einheiten ausgewählt ist.

**6.** Zusammensetzung nach einem der Ansprüche 1 - 5, wobei der Polyglycerylfettsäureester in einer Menge im Bereich von 0,5 Gew.-% bis 10 Gew.-%, vorzugsweise von 1 Gew.-% bis 5 Gew.-% und besonders bevorzugt von 1 Gew.-% bis 4 Gew.-%, bezogen auf das Gesamtgewicht der Zusammensetzung, vorhanden ist.

**7.** Zusammensetzung nach einem der Ansprüche 1 - 6, wobei der ethoxylierte/propoxylierte Fettalkohol in einer Menge im Bereich von 1 Gew.-% bis 10 Gew.-% und vorzugsweise von 2 Gew.-% bis 8 Gew.-%, bezogen auf das Gesamtgewicht der Zusammensetzung, vorhanden ist.

**8.** Zusammensetzung nach einem der Ansprüche 1 - 7, wobei Dipropylenglykol in einer Menge im Bereich von 5 Gew.-% bis 30 Gew.-%, vorzugsweise von 5 Gew.-% bis 20 Gew.-%, bezogen auf das Gesamtgewicht der Zusammensetzung, vorhanden ist.

**9.** Zusammensetzung nach einem der Ansprüche 1 - 8, wobei die Zusammensetzung ferner ein C3-C6-Polyol, bei dem es sich nicht um Dipropylenglykol handelt, umfasst, ausgewählt aus Glycerin, Propylenglykol, Butylenglykol, Pentylenglykol und Hexylenglykol.

**10.** Zusammensetzung nach Anspruch 9, wobei das C3-C6-Polyol, bei dem es sich nicht um Dipropylenglykol handelt, in einer Menge im Bereich von 1 Gew.-% bis 50 Gew.-%, vorzugsweise von 10 Gew.-% bis 40 Gew.-%, bezogen auf das Gesamtgewicht der Zusammensetzung, vorhanden ist.

**11.** Zusammensetzung nach Anspruch 1, die, bezogen auf das Gesamtgewicht der Zusammensetzung, Folgendes umfasst:

a) wenigstens einen kosmetischen Wirkstoff, ausgewählt aus Ferulasäure, Ascorbinsäure und Tocopherolen;
b) 1 Gew.-% bis 4 Gew.-% wenigstens eines nichtionischen Tensids, ausgewählt aus Polyglycerylcaprylat mit einer Polyglyceryl-Komponente, die von 2 bis 10 Glycerineinheiten abgeleitet ist, und ethoxylierten/propoxylierten C14-C22-Fettalkoholen mit 1-40 EO-Einheiten und 1-40 PO-Einheiten; und
c) 5 Gew.-% bis 20 Gew.-% Dipropylenglykol;

wobei die Zusammensetzung kein kationisches Tensid umfasst.

**12.** Nicht-therapeutisches Verfahren zur Pflege keratinhaltiger Materialien, umfassend:

i) Mischen der wasserfreien Zusammensetzung nach einem der Ansprüche 1 - 11 und von Wasser oder einem wasserhaltigen System, um eine Mikroemulsion auszubilden; und
ii) Auftragen der Mikroemulsion auf die keratinhaltigen Materialien.

**Revendications**

**1.** Composition anhydre destinée au soin des matières kératiniques comprenant :

a) au moins un principe actif cosmétique sensible à l'eau et/ou à l'oxygène choisi parmi l'acide férulique, l'acide ascorbique et les tocophérols ;
b) au moins un tensioactif non ionique choisi parmi les esters d'acides gras polyglycéryliques ayant une valeur HLB non inférieure à 13 à la température de 25 °C et les alcools gras éthoxylés/propoxylés ; et
c) du dipropylène glycol ;

dans laquelle la composition ne comprend aucun tensioactif cationique.

**2.** Composition selon la revendication 1, dans laquelle l'ester d'acide gras polyglycérylique est choisi parmi les mono-, di- et tri-esters de glycérine et un acide gras saturé ou insaturé comprenant 8 à 12 atomes de carbone.

**3.** Composition selon la revendication 1, dans laquelle l'ester d'acide gras polyglycérylique est choisi parmi le caprylate de polyglycéryle avec une fraction polyglycéryle dérivée de 2 à 10 motifs glycérol.

**4.** Composition selon l'une quelconque des revendications 1 à 3, dans laquelle l'alcool gras éthoxylé/propoxylé est choisi parmi les alcools gras en C14 à C22 éthoxylés/propoxylés.

**5.** Composition selon la revendication 4, dans laquelle l'alcool gras éthoxylé/propoxylé est choisi parmi les alcools gras en C14 à C22 éthoxylés/propoxylés ayant 1 à 40 motifs EO et 1 à 40 motifs PO.

**6.** Composition selon l'une quelconque des revendications 1 à 5, dans laquelle l'ester d'acide gras polyglycérylique est présent en une quantité comprise dans la plage allant de 0,5 % en poids à 10 % en poids, de préférence de 1 % en poids à 5 % en poids, et plus préférablement de 1 % en poids à 4 % en poids, par rapport au poids total de la composition.

**7.** Composition selon l'une quelconque des revendications 1 à 6, dans laquelle l'alcool gras éthoxylé/propoxylé est présent en une quantité comprise dans la plage allant de 1 % en poids à 10 % en poids, et de préférence de 2 % en poids à 8 % en poids, par rapport au poids total de la composition.

**8.** Composition selon l'une quelconque des revendications 1 à 7, dans laquelle le dipropylène glycol est présent en une quantité comprise dans la plage allant de 5 % en poids à 30 % en poids, de préférence de 5 % en poids à 20 % en poids, par rapport au poids total de la composition.

**9.** Composition selon l'une quelconque des revendications 1 à 8, dans laquelle la composition comprend en outre un polyol en C3 à C6 autre que le dipropylène glycol choisi parmi la glycérine, le propylène glycol, le butylène glycol, le pentylène glycol et l'hexylène glycol.

**10.** Composition selon la revendication 9, dans laquelle le polyol en C3 à C6 autre que le dipropylène glycol est présent en une quantité comprise dans la plage allant de 1 % en poids à 50 % en poids, de préférence de 10 % en poids à 40 % en poids, par rapport au poids total de la composition.

**11.** Composition selon la revendication 1, comprenant, par rapport au poids total de la composition :

a) au moins un principe actif cosmétique choisi parmi l'acide férulique, l'acide ascorbique et les tocophérols ;
b) de 1 % en poids à 4 % en poids d'au moins un tensioactif non ionique choisi parmi le caprylate de polyglycéryle avec une fraction polyglycéryle dérivée de 2 à 10 motifs glycérol et les alcools gras en C14 à C22 éthoxylés/-propoxylés avec 1 à 40 motifs EO et 1 à 40 motifs PO ; et
c) de 5 % en poids à 20 % en poids de dipropylène glycol ;

dans laquelle la composition ne comprend aucun tensioactif cationique.

**12.** Méthode non thérapeutique destinée au soin des matières kératiniques, comprenant :

i) le mélange de la composition anhydre selon l'une quelconque des revendications 1 à 11 avec de l'eau ou un système hydraté pour former une microémulsion ; et
ii) l'application de la microémulsion sur les matières kératiniques.

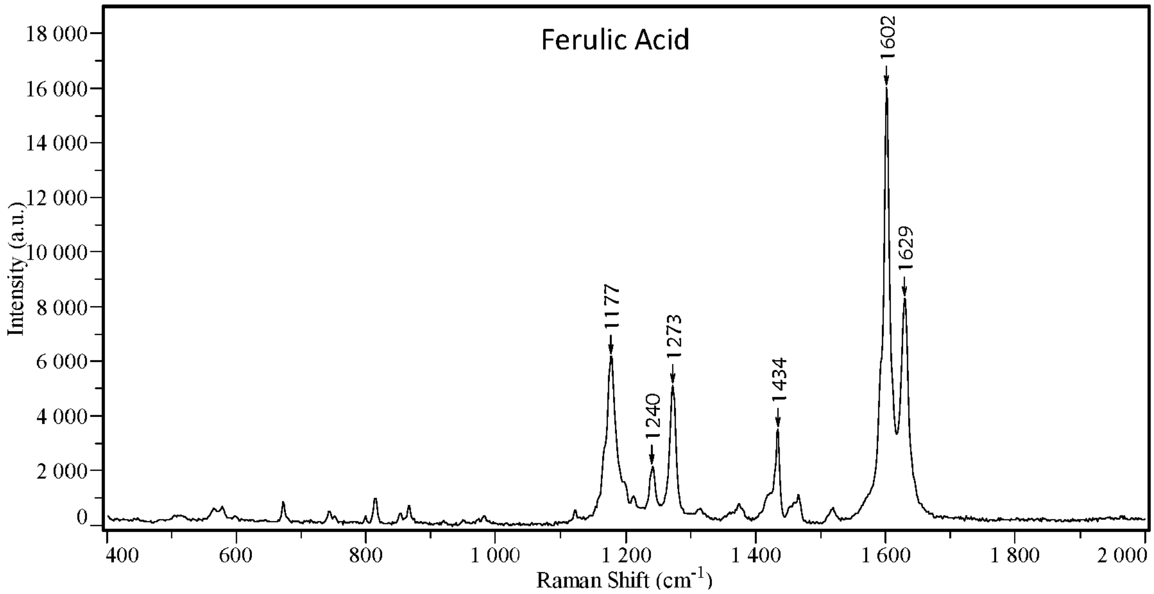
Ferulic Acid
Intensity (a.u.)
Raman Shift (cm-1)
18 000
16 000
14 000
12 000
10 000
8 000
6 000
4 000
2 000
0
400
600
800
1 000
1 200
1 400
1 600
1 800
2 000
1177
1240
1273
1434
1602
1629

Fig.1

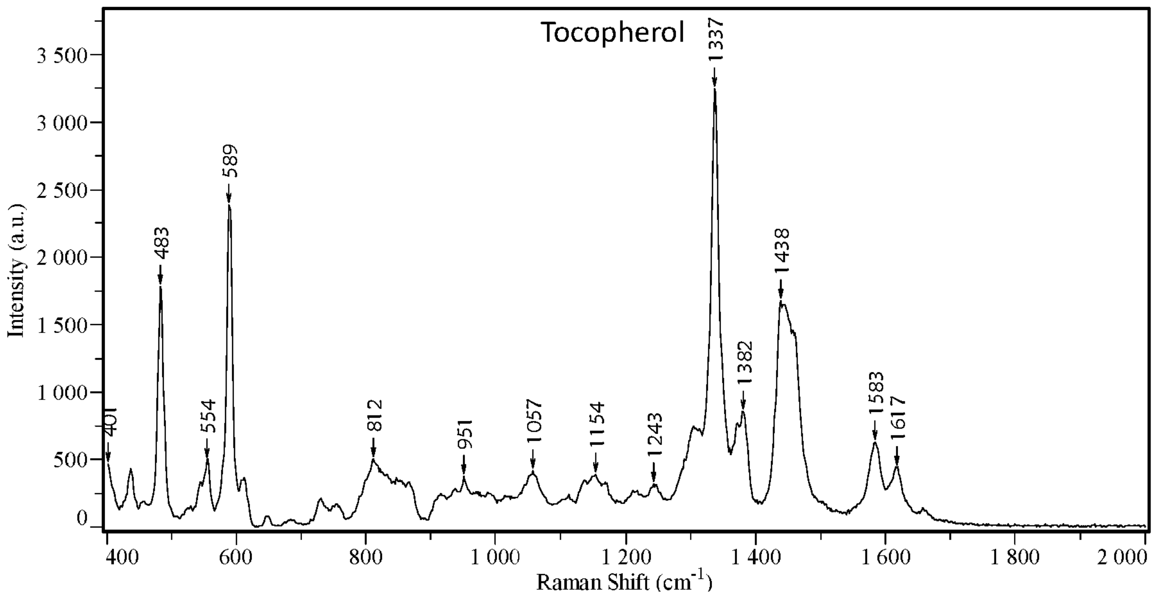
Tocopherol
Intensity (a.u.)
Raman Shift (cm-1)
3 500
3 000
2 500
2 000
1 500
1 000
500
0
400
600
800
1 000
1 200
1 400
1 600
1 800
2 000
401
483
554
589
812
951
1057
1154
1243
1337
1382
1438
1583
1617

Fig.2

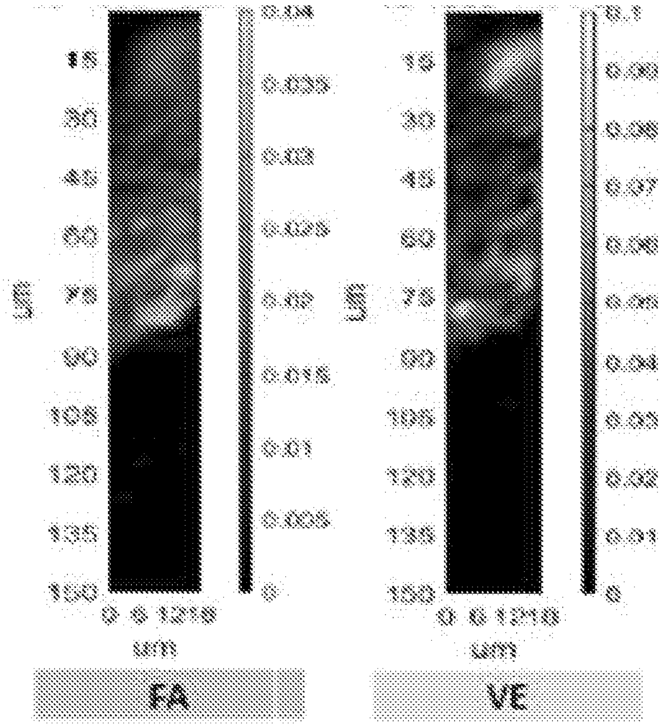

um
15
30
45
60
75
90
105
120
135
150
0 6 1218
um
0.04
0.035
0.03
0.025
0.02
0.015
0.01
0.005
0
FA
0.1
0.09
0.08
0.07
0.06
0.05
0.04
0.03
0.02
0.01
VE


Fig.3

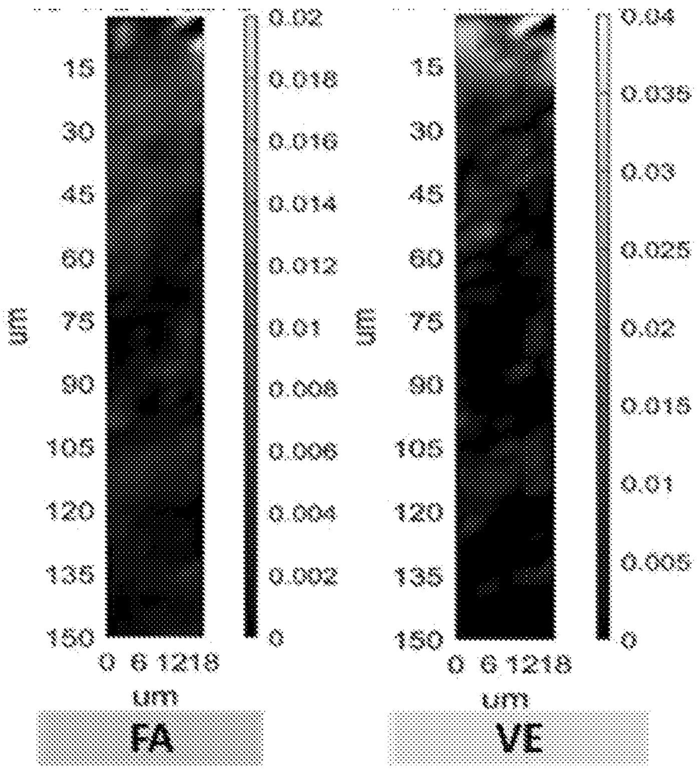

um
15
30
45
60
75
90
105
120
135
150
0 6 1218
um
0.02
0.018
0.016
0.014
0.012
0.01
0.008
0.006
0.004
0.002
0
FA
0.04
0.035
0.03
0.025
0.02
0.015
0.01
0.005
VE


Fig.4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0729746 A1


### Non-patent literature cited in the description


- **GRIFFIN**. *J. Ploughshare. Cosm. Chem.*, 1954, vol. 5, 249-256
- **SATOSHI TOMOMASA et al.** *Oil Chemistry*, 1988, vol. 37 (11), 48-53